Europäisches Patentamt

European Patent Office

Office européen des brevets

(11) Publication number: **0 290 261 B1**

(12) **EUROPEAN PATENT SPECIFICATION**

(45) Date of publication of patent specification: **23.06.93**

(51) Int. Cl.5: **C12N 15/85**, C12N 9/48, A61K 37/02

(21) Application number: **88304093.3**

(22) Date of filing: **06.05.88**

The file contains technical information submitted after the application was filed and not included in this specification

(54) **Expression of foreign genes in drosophila cells.**

(30) Priority: **08.05.87 US 47736**

(43) Date of publication of application:
**09.11.88 Bulletin 88/45**

(45) Publication of the grant of the patent:
**23.06.93 Bulletin 93/25**

(84) Designated Contracting States:
**AT BE CH DE ES FR GB GR IT LI LU NL SE**

(56) References cited:
**EP-A- 0 093 619
EP-A- 0 117 059
EP-A- 0 211 260
EP-A- 0 248 647**

**Proceedings of the National Academy of Sciences of the United States of America, vol. 80, no.23, December 1983 (Baltimore, USA). P.P. Di Nocera et al."Transient expression of genes introduced into cultured cells of Drosophila", pages 7095-7098**

**T.A. Bunch and L. Goldstein, 26th Annual Meeting of the American Society for Cell Biology, in J. Cell Biol. 5, part 2, 1986**

Nucleic Acid Research, 1988, vol. 16, pp. 1043-1061

(73) Proprietor: **SMITHKLINE BEECHAM CORPORATION**
**One Franklin Plaza P.O. Box 7929**
**Philadelphia Pennsylvania 19103(US)**

(72) Inventor: **Johansen, Hanne Ranch**
**649 South Henderson Road**
**King of Prussia Pennsylvania 19406(US)**
Inventor: **van der Straten-Ponthoz, Ariane Adrienne**
**1911 Rodman Street**
**Philadelphia Pennsylvania 19146(US)**
Inventor: **Rosenberg, Martin**
**241 Mingo Road**
**Royersford Pennsylvania 19468(US)**

(74) Representative: **Valentine, Jill Barbara et al**
**SmithKline Beecham Corporate Patents**
**Great Burgh Yew Tree Bottom Road**
**Epsom Surrey KT18 5XO (GB)**

## Description

Field of Invention

This invention relates to a method for the expression of foreign genes in Drosophila cells. More specifically it relates to a system for expression of high levels of tissue plasminogen activator (tPA) in Drosophila cells and purification of the expressed gene product.

Background of the Invention

Human tPA is a glycoprotein of about 70,000 daltons and 527 amino acids. It is secreted by a variety of human tissues and is naturally present in serum. tPA functions in clot lysis by converting plasminogen to plasmin. tPA has an affinity for and is more active in the presence of fibrin. Thus, tPA is useful in treating disorders associated with thrombi formation such as deep vein thrombosis, acute myocardial infarction and pulmonary embolism. See, generally, Rijken et al., Biochem. Biophys. Acta 580:140 (1979), Vetterlein et al., J. Biol. Chem. 254:575 (1979), Rijken et al., J. Biol. Chem. 256:7035 (1981) and Matsuo et al., Nature 291:590 (1981).

tPA is overproduced by certain cell lines, including the Bowes melanoma cell line. See, e.g., Wilson et al.,Canc. Res. 40:933 (1980). Collen et al., European Patent Application (EP-A-41,766), disclose purification of Bowes melanoma-derived tPA. Wilson, EP-A-113,319, disclose production and purification of tPA using a serum-independent Bowes melanoma cell line. Brouty-Boye, Bio/Technology, December 1984, p. 1058, reports production of tPA by human embryonic lung cells. Schleuning et al., Sixth Int'l Conf. Fibrinolysis, Lausanne, Switzerland, Abstract, July 22-23, 1982 report production of tPA by HeLa cells.

tPA can also be prepared by recombinant DNA techniques. Isolation of mRNA for tPA is disclosed, e.g., by Opdenakker et al., Eur. J. Biochem. 121:269 (1982). Isolation of cDNA for part of tPA is disclosed by Edlund et al., Proc. Natl. Acad. Sci. USA 80:349 (1983). Cloning of cDNA for tPA in E. coli is reported by Pennica et al., Nature 301:214 (1983). Cloning of cDNA for tPA in E. coli and in Chinese Hamster Ovary cells by application of routine recombinant DNA procedures is disclosed by Goeddel et al., EP-A-93,619 and by Levinson et al., EP-A-117,059. Goldberg et al., PCT patent application WO85-03949, disclose expression of tPA in E. coli. Meyhack et al., EP-A-143,081, disclose expression of tPA in yeast. Robinson, WO84-01786, discloses a modified tPA lacking all or a portion of the carbohydrate moieties present in native tPA.

The development of Drosophila cell cultures which are stable and can be grown under laboratory conditions have been reported. See Schneider, J. Embryol. Exp. Morphol. 27:353 (1972). Various vectors systems containing specific coding sequences have been inserted into Drosophila under the control of the Drosophila heat shock promoter or COPIA promoters. DiNocera et al., Proc. Natl. Acad. Sci. USA 80:7095 (1983). More recently, mRNA encoding the heat-shock promoters has been translated in Drosophila cells at high rates, McGarry et al., Cell 42:903 (1985).

Summary of Invention

One embodiment of the present invention relates to the development of a selection system to co-introduce and express foreign genes in Drosophila cells. By use of this method, tPA can be produced in Drosophila cells by culturing Drosophila cells transfected with a tPA gene expression unit such that the tPA is expressed and secreted and collecting the secreted tPA. The selection system developed can be used to co-introduce and overexpress other genes of interest.

Another embodiment of the invention relates to tPA produced by a recombinant Drosophila cell. A tPA gene expression unit comprising a DNA coding sequence for tPA, or an active variant thereof, and a regulatory element necessary for the transcription of the tPA coding sequence and subsequent translation within a Drosophila cell is described. The tPA gene product is then secreted into the cell media and collected.

In another embodiment, the invention is a method for expressing a heterologous gene product in Drosophila which comprises cotransfecting Drosophila cells with a vector containing a gene expression unit for the gene product and a vector containing the 5'LTR from an integrated COPIA element and a hygromycin B phosphotransferase or methotrexate resistance selection marker; culturing transfected cells under conditions such that the gene product is expressed; and collecting the gene product.

Detailed Description of the Invention

It has now been found that high level production of tPA can be obtained in a Drosophila cell culture. The Drosophila cells are transfected by using standard cloning techniques which permit introduction of foreign DNA into a host cell without adversely affecting the foreign DNA or the host cell. The recombinant Drosophila cells so constructed secrete tPA at high levels.

Any Drosophila cells can be used in the practice of the invention. A preferred cell line is the $S_2$ line. Introduction of the tPA cDNA coding sequence into Drosophila $S_2$ cells by DNA transfection techniques produces unexpectedly large amounts of tPA. The $S_2$ cells (Schneider, J. Embryol. exp. Morph. 27:353 (1972)) are stable cell cultures of polyploid embryonic Drosophila cells. Use of the $S_2$ Drosophila cell has many advantages including but not limited to its ability to grow to a high density at room temperature. Stable integration of the selection system has produced up to 1000 copies of the transfected gene expression unit into the cell chromosomes. Other available Drosophila cell lines are $S_1$, $S_3$, KC-O and hydei.

Drosophila cells can be cultured in a variety of nutrient media, including, e.g. $M_3$ media. The $M_3$ media consists of a formulation of balanced salts and essential amino acids at a pH of 6.6. Preparation of the media is substantially as described by Lindquist, DIS, 58:163 (1982).

A tPA gene expression unit which is a recombinant DNA molecule and which can be used to prepare the recombinant Drosophila cells of the invention comprises a DNA coding sequence for tPA, or for an active variant thereof, and regulatory regions necessary or desirable for transcription of the tPA coding sequence and subsequent translation.

The tPA coding sequence is available from several sources or can be obtained from human cells or cell lines, such as the Bowes melanoma cell line or the HeLa cell line. Standard techniques of genetic engineering are utilized to derive a tPA coding sequence from the genomic DNA of a human cell. Variants thereof can also be prepared by standard recombinant DNA techniques. Such variants comprise tPA molecules in which one or a few amino acids have been substituted, added or deleted without significantly adversely affecting thrombolytic activity. See, for example, Robinson, WO84-01786; Rosa et al., WO86-01538; Heyneker et al., GB2,173,804A.

The regulatory region typically comprises a promoter region which functions in the binding of RNA polymerase and in RNA transcription initiation. The promoter region is typically found upstream from the tPA coding sequence. Promoters which are commonly employed in mammalian cell expression vectors including, e.g., avian Rous sarcoma virus LTR and simian virus (SV40 early promoter) demonstrate poor function and expression in the Drosophila system. The preferred promoters are of Drosophila origin. It has been found that the SV40 early promoter gives lower levels of expression than the Drosophila metallothionein promoter. Examples of other Drosophila promoters include, e.g., heatshock (Hsp70) and COPIA LTR promoters.

The tPA coding sequence is followed by a polyadenylation (poly A) region within the tPA gene expression unit, such as an SV40 early poly A region. The poly A region which functions in the polyadenylation of RNA transcripts appears to play a role in stabilizing transcription. The poly A region can be derived from a variety of genes in which it is naturally present. Other available poly A regions include, e.g., those derived from growth hormone and metallothionein genes. Such region can also be modified to alter its sequence provided that polyadenylation and transcript stabilization functions are not significantly adversely affected.

Use of the Drosophila metallothionein promoter results in the metallothionein vector system retaining full regulation even at very high copy number. This is in direct contrast to the results in mammalian cells using the mammalian metallothionein promoter wherein the regulatory effect of the metal is diminished as copy number increases. The result of this retained inducibility effect is increased expression of the gene product in the Drosophila cell at high copy number.

Once the vector containing the tPA gene expression unit has been constructed, it can be transfected into the Drosophila cell using standard transfection techniques. Such techniques include, for example, calcium phosphate co-precipitation, cell fusion, electroporation, microinjection and viral transfection.

It is typical when doing gene cloning to incorporate the gene of choice into a larger recombinant DNA molecule prior to transfection. Such larger DNA molecule preferably comprises at least a genetic selection marker in addition to the tPA functions. The selection marker can be any gene or genes which cause a readily detectable phenotypic change in a transfected host cell. Such phenotypic change can be, for example drug resistance, such as the gene for hygromycin B resistance.

A selection system using the drug methotrexate and procaryotic dihydrofolate reductase (DHFR) can be used with Drosophila cells. The endogenous eucaryotic DHFR of the cells is inhibited by methotrexate. Therefore, by transfecting the cells with a plasmid containing the procaryotic DHFR which is insensitive to methotrexate and selection with methotrexate, only cells transfected with and expressing the procaryotic DHFR will survive.

Unlike methotrexate selection of transformed mammalian and bacterial cells, in the Drosophila system, methotrexate can be used to initially select high copy number transfectants. Only cells which have incorporated the protective procaryotic DHFR gene will survive. Concomitantly, these cells have the gene expression unit of interest.

A two vector system can be used in the present invention to cotransfect into the Drosophila cell a gene expression unit for a gene product of interest and the coding region for the selection system to be used. For example, the pDM100 expression system, consisting of the vectors pDM100 and pHGCO, can be used to prepare recombinant $S_2$ cells containing the DNA coding sequence for tPA. The vector, pDM100, comprises a tPA gene expression unit in which the promoter is the Hsp70 heatshock promoter from Drosophila. Hsp 70 is a Drosophila heatshock promoter which has been found advantageous for gene expression in Drosophila cells (Ingolia, et al., Cell, 21:669 (1980)). The pHGCO vector comprises a DHFR gene expression unit and is cotransfected with the pDM100 vector thereby providing the DHFR gene necessary for selection. A more complete description of this embodiment of the invention is found in Example 1.

A second illustrative embodiment of this invention is the production of tPA using a two vector method but with a tPA gene expression unit which employs the pCOHYGRO vector system consisting of the vectors pCOHYGRO and pDMtPA.

pCOHYGRO comprises a hygromycin B gene expression unit. pDMtPA contains the tPA gene expression unit using the metallothionein promoter. A tPA gene expression unit which utilizes the pCOHYGRO vector system will produce tPA in $S_2$ Drosophila cells by maximizing the advantage of hygromycin B resistance for selection. With this system, the antibiotic hygromycin B can be used to select for those cells containing the transfected vectors.

The two vectors are cotransfected into the $S_2$ Drosophila cell using the method as described by Wigler et al., Cell 16:777 (1979). The vectors are cotransfected in varying ratios depending upon the particular copy number desired. The transfected cells are then selected, such as in $M_3$ medium containing serum and the appropriate selection agent, e.g. hygromycin B or methotrexate.

Other known Drosophila cell culture systems which may be used are, for example, the KC-O Drosophila melanogaster cell line which is a serum free cell line, Schulz, et al., Proc. Natl. Acad. Sci. USA 83:9428 (1986). Preliminary studies using the KC-O line have suggested that transfection is more difficult than with $S_2$ cells. Another cell line which has been used is a cell line from Drosophila hydei. Protein expression can be obtained using the hydei cell line, however, transfection into this cell line can result in the transfected DNA being expressed with very low efficiency, Sinclair, et al., Mol. Cell. Biol. 5:3208 (1985).

Once an appropriate vector system has been constructed and transfected into the $S_2$ cell line, the expression of tPA is induced by the addition of an appropriate inducing agent, such as cadmium in the case of the metallothionein promoter or heat in the case of the Hsp70 promoter. Transcription of the tPA coding sequence after induction can be monitored.

Southern blot analysis. (Maniatis, et al., Molecular Cloning, Cold Spring Harbor Laboratory, Cold Spring Harbor, N.Y. 1982) can be used to determine copy number of the tPA gene. Northern blot analysis (Maniatis, supra, p. 202) provides information regarding the size of the transcribed gene sequence. The level of transcription can also be quantitated. A specific tPA mRNA of approximately 2.4 kb can be determined. Expression of tPA in the recombinant cells can be further verified through Western blot analysis and activity tests on the resulting protein.

The $S_2$ cells of the present invention are especially suited to high yield production of protein. The cells can be maintained in suspension cultures at room temperature (24±1°C). Culture medium is $M_3$ supplemented with between 5 and 10% (v/v) heat inactivated fetal bovine serum. In the preferred embodiment of the invention the culture medium contains 5% fetal bovine serum. After induction, the cells are cultured in serum free media. When the pCOHYGRO vector system is used, the media is also supplemented with 300 $\mu$g/ml hygromycin B. In this media the $S_2$ cells can be grown in suspension cultures, for example in 250 ml to 2000 ml spinner flasks, with stirring at 50-60 rpm. Cell densities are typically maintained between $10^6$ and $10^7$ cells per ml. In one embodiment of this invention the cells are grown prior to induction in 1500 ml spinner flasks in media containing 5% serum.

Following cell culture, the tPA can be isolated from the spent media such as by use of a monoclonal antibody affinity column. Other known protein purification steps, e.g. metal chelates, various affinity chromatography steps or adsorption chromatography, can be used to purify the tPA from the culture media. The use of the cell line $S_2$ which secretes the gene product directly into the media is an important feature of the present invention. Direct secretion into the media allows utilization of an efficient one-step purification system. Using a monoclonal antibody column directed against tPA, the spent culture media can be added directly to the column and the tPA eluted using 1.5 M KSCN in phosphate buffered saline.

The Drosophila cell system has been used to produce entirely single chain tPA. The ratio of long to short form at the amino-terminus is found to be completely opposite to that found in mammalian systems. Preliminary gel mobility studies indicate the Drosophila tPA also differs in glycosylation. Because the human tPA is being produced in an insect system, the tPA is completely free of human, hamster or mouse contaminating materials. In addition, human tPA made in Drosophila is free of mammalian retroviruses.

Following additional purification methods in order to obtain pharmaceutical grade tPA, the tPA is formulated into a pharmaceutical composition (European Patent Application 0,211,592) such that each dosage unit comprises an amount of tPA which is non-toxic, that is, free of significant adverse side effects, and which is effective in causing thrombolysis following administration to an animal, particularly man. Such pharmaceutical composition for treatment of myocardial infarction typically is in the form suitable for intravenous injection or infusion and comprises 0.1 to 10 mg/ml of tPA in a pharmaceutically acceptable diluent or carrier. Suitable diluents and carriers are well known to a pharmaceutical chemist, as are techniques for preparing the pharmaceutical composition.

Although the preceding description and the examples which follow are directed primarily to expression of tPA in Drosophila, the expression vectors and the two vector expression systems described herein can be used to clone and express other genes and coding sequences in Drosophila. For example, a coding sequence for a growth hormone, a lymphokine, urokinase, an antigen or other gene product of interest can be inserted within a gene expression unit into a vector such as pDM100 or pDMtPA and cotransfected with a vector such as pHGCO or pCOHYGRO. Transfected cells can then be selected as described above.

Examples

The examples which follow are illustrative, and not limiting of the invention.

EXAMPLE 1

Expression of tPA using the Hsp70 vector and a DHFR selection system.

Construction of Vectors:

pDMKΔH

As the basic vector for gene expression in Drosophila pML2 was used, which is a small pBR322 vector containing the β-lactamase gene (Mellon et al., Cell 27:297 (1982)). This vector was digested with Sal I which creates a unique cut in the plasmid. Into this site was inserted a Sal I casette containing the SV40 early promoter followed by the galactokinase gene and the SV40 early polyadenylation site.

The Sal I casette was obtained from pDSPI (Pfarr et al., DNA 4:461 (1985)). The orientation of the insert was determined by fine restriction analysis using HindIII and BamHI. The new construct pDMK has the transcription unit for the galactokinase and β-lactamase gene running in the opposite direction. pDMKΔH was created by a deletion of a HindIII site in the pBR322 sequence 5′ to the SV40 early promoter. pDMK was digested with HindIII and the site was endfilled (Maniatis, supra p. 113) and the vector was religated. pDMKΔH now has a unique HindIII site positioned between the SV40 early promoter and the galactokinase gene.

pDMKHSP

pDMKΔH was digested with SmaI and HindIII which drops out the SV40 early promoter. The HindIII site was end-filled and the Drosophila HSP70 promoter fragment was inserted into this vector.

The fragment used was a 460 bp fragment obtained by XbaI and XmnI digestion of pDM301 (McGarry, et al., Cell 42:903 (1985)). This fragment contains what is thought to be the regulatory sequences for heat induction. The fragment was endfilled and ligated to the SmaI, HindIII cut vector.

The ligated DNA was transformed into E. coli and colonies containing the desired plasmid were identified by fine restriction of minipreparations of DNA with HindIII and XhoI.

5

pDM100

pDMKHSP was cut with XbaI, then endfilled and finally cut with HindIII. This drops out the galactokinase gene. The tPA coding sequence was isolated from a cDNA clone obtained from Schleuning, et al., supra, on a HindIII-BalI fragment which includes the prepropeptide and the entire coding sequence of tPA. This fragment was inserted into the cut pDMKHSP vector. The ligated DNA was transformed into E. coli and the correct plasmid was identified by restriction with the enzymes HindIII, BamHI and XbaI. Conditions for all restrictions were as recommended by manufacturer (New England Biolabs).

Transfection into Drosophila $S_2$ Cells

pDM100 was cotransfected into $S_2$ Drosophila cells together with pHGCO which is a plasmid containing the E. coli derived procaryotic DHFR gene driven by COPIA 5′LTR (Bourouis et al., EMBO 2:1104 (1983)). The transfection procedure was substantially as described by Wigler et al., Cell 16:777 (1979). 20μg plasmid DNA pDM100 + pHGCO was cotransfected in varying ratios depending upon the copy number desired into $3 \times 10^6$ cells and the precipitate was left on the cells for 18 hours. The cells were then washed and allowed to grow 48 hours to allow expression of DHFR. Cells were spun down and resuspended in medium containing $2 \times 10^{-7}$ M methotrexate to select for transformants expressing the DHFR gene. Stable transformants were obtained after 6 weeks selection.

Analysis of gene product

Resistant cells were seeded at a density of $5 \times 10^6$ cells/ml in $M_3$ medium without serum. The cells were allowed to express tPA for 3 days before the media was collected and assayed for the presence of tPA protein. Western analysis substantially as described by Bittner, et al., Ann. Biochem. 102:549 (1980) showed the presence of high amounts of tPA protein, i.e. the protein has the expected molecular weight (approx. 70 kd) for single chain tPA. Confirmation that the tPA protein was expressed and active was obtained using the S2251 activity assay. Ranby, et al., Thromb. Res. 27:743 (1982).

Southern analysis of the tPA producing polyclonal cells showed that they contained up to 1000 copies of the tPA gene expression unit per cell and that the tPA gene expression unit is amplified and integrated in the host chromosomes in a head to tail pattern.

EXAMPLE 2

Expression of tPA using the hygromycin B selection system.

Construction of vectors:

pUCOPIA

As the basic cloning vector for gene expression in Drosophila, pUC18 containing a BamHI and SmaI site was used. The 5′LTR from an integrated COPIA element (357 base pairs) was cloned into the BamHI site of vector pUC18 resulting in the vector designated pUCOPIA. COPIA is a representative member of the disperse middle repetitive sequences found scattered through the Drosophila genome (Rubin, et al. in Cold Spring Harbor Symp. Quant. Biol. 45:619 (1980)).

pCOHYGRO

The vector pUCOPIA was cut at the SmaI site and the E. coli gene coding for hygromycin B phosphotransferase (hygromycin B cassette) was cloned into pUCOPIA using standard cloning techniques. The hygromycin B cassette was isolated on a HindIII - BamHI fragment of 1481 base pairs from the vector DSP-hygro (Gertz, et al. Gene 25:179 (1983)). The hygromycin B cassette contains the sequence coding for the hygromycin B phosphotranferase gene and the SV40 early poly A region. The Hind III and BamHI sites were filled in using $T_4$ DNA polymerase and the hygromycin B cassette was ligated into the SmaI site of the vector pUCOPIA.

pDMtPA

The plasmid pBR322 containing the β-lactamase gene was used as the basic vector. The metallothionein promoter, Lastowski-Perry, et al., J. Biol. Chem. 260:1527 (1985) was isolated on an EcoR1-StuI fragment and was inserted into pBR322 in front of the entire tPA gene containing the prepeptide. An SV40 early polyA site follows the tPA gene.

Transfection into Drisophila $S_2$ cells:

pCOHYGRO was cotransfected into $S_2$ Drosophila cells together with pDMtPA carrying the tPA gene under the control of the Drosophila metallothionein promoter. The transfected cells were selected in $M_3$ medium with serum containing 300 μg/ml of hygromycin B. After 2 to 3 days under identical conditions the untransfected cells stop dividing and begin to die. The time of selection in order to obtain stable, growing hygromycin B resistant cells in the transfected cultures is approximately two to three weeks.

To obtain cultures having integrated into their chromosomes different copy numbers of the tPA gene, the ratios of the two vectors were varied at transfection as indicated in Table 1.

## Table 1

| pDMtPA | pCOHYGRO | Isolated polyclonal Culture | Approx. Copy Number | |
| --- | --- | --- | --- | --- |
| | | | Hygromycin B Gene | tPA Gene |
| 10mg | 10mg | A | 50-100 | 30-40 |
| 18mg | 2mg | B | 50-100 | 150 |
| 20mg | 0.2mg | C | 50-100 | 2000 |

Expression of tPA was verified after induction of the metallothionein promoter with 10 μM cadmium. A specific tPA mRNA of approximately 2.4 kb was detected by Northern blot analysis. Concomitantly, Western blot analysis of the spent supernatant from the induced cell cultures revealed a single tPA band at approximately 70 kd.

The level of tPA in the cell supernatants was measured using the S2251 Activity Test. $5 \times 10^6$ cells were seeded in 1 ml of $M_3$ medium without serum and induced for 3 to 4 days. The level of tPA measured in the supernatant is indicated in Table 2.

## Table 2

| polyclonal culture | uninduced cells (ng/ml) | induced cells (ng/ml) |
| --- | --- | --- |
| A | 160 | 500 |
| B | 99 | 1500 |
| C | 85 | 1300 |
| control pCOHYGRO | 0 | 0 |

Scale up of polyclonal culture B along with two single clone cultures derived from polyclonal culture B using standard soft-agar cloning techniques resulted in significant increase in tPA production as shown in Table 3.

7

### Table 3

| Cell Culture | Copy Number | µg/ml (after 3 days) |
|---|---|---|
| polyclone culture B | 100-150 | 14-20 |
| single clone T | 30 | 3-12 |
| single clone I | 150 | 22-37 |

Cells were maintained as suspension cultures in 250 ml to 2000 ml spinner flasks. Culture medium was M3 supplemented with 300 µg/ml hygromycin B. Cultures were incubated at 24±1°C and stirred at 50-60 rpm. Cell densities were typically maintained between $10^6$ and $10^7$ cells per ml.

For induction, cells were harvested by low-speed centrifugation and resuspended at a density of 5 x $10^6$ cells per ml in $M_3$ medium supplemented with hygromycin B. Cadmium chloride was added to a final concentration of 10µM, and the cultures were allowed to grow for 3 to 4 days in serum free media prior to harvesting the tPA.

Example 3

Purification of tPA from conditioned cell culture media

PAM-2 Sepharose® (American Diagnostica, Greenwich, Ct.) is tPA monoclonal antibody coupled to Sepharose 4B. 5 ml of PAM-2 Sepharose (approximately 10 mg monoclonal tPA antibody per column volume) was transferred to a 1 x 6 cm column and washed with 2M KSCN at 5 ml/hr. overnight to remove loosely bound monoclonal antibody. After washing, 50 ml of phosphate buffered saline (PBS) was passed through the column to equilibrate the gel.

The sterile filtered conditioned media from the induced Drosophila cells (1 liter) was slowly passed through the column at 0.8 ml/min. The column was then washed with 30 ml of PBS followed by 30 ml 0.5 M KSCN in PBS at 0.8 ml/min. The tPA was eluted using 30 ml of 1.5 M KSCN followed by 2.0 M KSCN at a flow rate of 0.8 ml/min. Greater than 90% of the tPA eluted in a single peak with 1.5 M KSCN. Following the elution with 2.0 M KSCN in order to elute any residual, tightly binding tPA, the column was washed with 30ml PBS then stored in 0.01% Sodium Azide in PBS.

An analysis of the tPA produced in the Drosophila cells showed a direct correlation between the cell density at time of induction and the amount of tPA produced. It was found that induction of cells at a density 5 x $10^6$ greatly increased the concentration of tPA being secreted into the media.

Assays

The tPA concentration was determined using various standard assays including HPLC, the S2251 Assay, the particle fluorescence immunoassay (PCFIA) performed according to the protocol provided by the manufacturer (Pandex Laboratories, Inc. Mundelein, Ill.), and the enzyme immuno-assay (EIA) performed substantially as described in the protocol provided by the manufacturer (American Diagnostica).

Using the S2251 assay, the activity of the purified tPA has been found to be at least the same as the highest reported for expression in mammalian cells.

The above description and examples fully disclose the invention including preferred embodiments thereof. Modifications of the methods described that are obvious to those of ordinary skill in molecular genetics and related sciences are intended to be within the scope of the following claims.

**Claims**
**Claims for the following Contracting States : AT, BE, CH, DE, FR, GB, IT, LI, LU, NL, SE, GR**

**1.** A method for expressing a non-bacterial heterologous gene product in Drosophila cells which comprises transfecting said cells with a gene expression unit for the heterologous gene product and a selection marker; culturing transfected cells under conditions such that the gene product is expressed; and collecting the gene product.

2. The method of claim 1 wherein the promoter in the gene expression unit is a Drosophila metallothionein promoter.

3. The method of any preceding claim wherein the selection marker is hygromycin B phosphotransferase or dihydrofolate reductase (DHFR).

4. The method of any preceding claim wherein the gene expression unit for the heterologous gene product and the selection marker are in separate vectors with which the Drosophila cells are cotransfected.

5. The method of any preceding claim wherein the heterologous gene product is a protein encoded by a DNA coding sequence for tPA or an active variant thereof.

6. A method for expressing a heterologous gene product in Drosophila cells which comprises transfecting cells with a gene expression unit for the heterologous gene product and a selection marker, wherein the selection marker is hygromycin B phosphotransferase; culturing transfected cells under conditions such that the gene product is expressed; and collecting the gene product.

7. The method of claim 6 wherein the promoter in the gene expression unit for the heterologous gene product is a Drosophila promoter.

8. The method of claim 7 wherein the promoter is the metallothionein promoter, the Hsp-70 promoter or the 5' LTR of a COPIA element.

9. The method of any one of claims 6 to 8 wherein the gene expression unit for the heterologous gene product and the selection marker are in separate vectors with which the Drosophila cells are cotransfected.

10. The method of any one of claims 6 to 9 wherein the heterologous gene product is a protein encoded by a DNA coding sequence for tPA or an active variant thereof.

11. A method for production of a protein encoded by a DNA coding sequence for tPA or an active variant thereof in Drosophila cells which comprises cotransfecting cells with a vector containing a gene expression unit for tPA and a vector containing a selectable marker, culturing transfected cells under conditions such that the tPA is expressed and secreted and collecting the secreted tPA.

12. A tPA gene expression unit comprising a DNA coding sequence for tPA, or an active variant thereof, and a regulatory element which functions in Drosophila cells necessary for transcription of the tPA coding sequence and subsequent translation within a Drosophila cell.

13. A method for expressing a heterologous gene product in Drosophila cells which comprises cotransfecting said cells with a gene expression unit for the heterologous gene product and a DHFR selection marker; culturing the transfected cells whereby the DHFR gene and the heterologous gene expression unit are expressed without further amplification; and collecting the heterologous gene product.

14. The method of claim 13 wherein the promoter in the gene expression unit for the heterologous gene product is a Drosophila promoter.

15. The method of claim 14 wherein the promoter is the metallothionein promoter, the Hsp-70 promoter or the 5' LTR of a COPIA element.

16. A protein encoded by a DNA coding sequence for tPA or an active variant thereof obtainable by the method of any one of claims 1,6,11 and 13.

17. A pharmaceutical composition for effecting thrombolysis in a human subject which comprises an effective, non-toxic amount of the tPA of claim 16 and a pharmaceutical carrier thereof.

**Claims for the following Contracting State : ES**

1. A method for expressing a non-bacterial heterologous gene product in Drosophila cells which comprises transfecting said cells with a gene expression unit for the heterologous gene product and a selection marker; culturing transfected cells under conditions such that the gene product is expressed; and collecting the gene product.

2. The method of claim 1 wherein the promoter in the gene expression unit is a Drosophila metallothionein promoter.

3. The method of any preceding claim wherein the selection marker is hygromycin B phosphotransferase or dihydrofolate reductase (DHFR).

4. The method of any preceding claim wherein the gene expression unit for the heterologous gene product and the selection marker are in separate vectors with which the Drosophila cells are cotransfected.

5. The method of any preceding claim wherein the heterologous gene product is a protein encoded by a DNA coding sequence for tPA or an active variant thereof.

6. A method for expressing a heterologous gene product in Drosophila cells which comprises transfecting said cells with a gene expression unit for the heterologous gene product and a selection marker, wherein the selection marker is hygromycin B phosphotransferase; culturing transfected cells under conditions such that the gene product is expressed; and collecting the gene product.

7. The method of claim 6 wherein the promoter in the gene expression unit for the heterologous gene product is a Drosophila promoter.

8. The method of claim 7 wherein the promoter is the metallothionein promoter, the Hsp-70 promoter or the 5' LTR of a COPIA element.

9. The method of any one of claims 6 to 8 wherein the gene expression unit for the heterologous gene product and the selection marker are in separate vectors with which the Drosophila cells are contransfected.

10. The method of any one of claims 6 to 9 wherein the heterologous gene product is a protein encoded by a DNA coding sequence for tPA or an active variant thereof.

11. A method for production of a protein encoded by a DNA coding sequence for tPA or an active variant thereof in Drosophila cells which comprises cotransfecting cells with a vector containing a gene expression unit for tPA and a vector containing a selectable marker, culturing transfected cells under conditions such that the tPA is expressed and secreted and collecting the secreted tPA.

12. A method for expressing a heterologous gene product in Drosophila cells which comprises cotransfecting said cells with a gene expression unit for the heterologous gene product and a DHFR selection marker; culturing the transfected cells whereby the DHFR gene and the heterologous gene expression unit are expressed without further amplification; and collecting the heterologous gene product.

13. The method of claim 12 wherein the promoter in the gene expression unit for the heterologous gene product is a Drosophila promoter.

14. The method of claim 13 wherein the promoter is the metallothionein promoter, the Hsp-70 promoter or the 5' LTR of a COPIA element.

**Patentansprüche**
**Patentansprüche für folgende Vertragsstaaten : AT, BE, CH, DE, FR, GB, IT, LI, LU, NL, SE, GR**

1. Verfahren zur Expression eines nicht-bakteriellen, heterologen Genprodukts in Drosophila-Zellen, bei dem man die Zellen mit einer Genexpressions-Einheit für das heterologe Genprodukt und einem Selektionsmarker transfiziert; die transfizierten Zellen unter Bedingungen züchtet, unter denen das Genprodukt exprimiert wird; und das Genprodukt sammelt.

2. Verfahren nach Anspruch 1, wobei der Promotor der Genexpressions-Einheit ein Drosophila-Metallothionein-Promotor ist.

3. Verfahren nach einem der vorhergehenden Ansprüche, wobei der Selektionsmarker die Hygromycin B-Phosphotransferase oder die Dihydrofolatreduktase (DHFR) ist.

4. Verfahren nach einem der vorhergehenden Ansprüche, wobei die Genexpressions-Einheit für das heterologe Genprodukt und der Selektionsmarker in getrennten Vektoren liegen, mit denen die Drosophila-Zellen cotransfiziert werden.

5. Verfahren nach einem der vorhergehenden Ansprüche, wobei das heterologe Genprodukt ein Protein, das von einer für tPA codierenden DNA-Sequenz codiert wird, oder eine aktive Variante davon ist.

6. Verfahren zur Expression eines heterologen Genprodukts in Drosophila-Zellen, bei dem man Zellen mit einer Genexpressions-Einheit für das heterologe Genprodukt und einem Selektionsmarker transfiziert, wobei der Selektionsmarker die Hygromycin B-Phosphotransferase ist; die transfizierten Zellen unter Bedingungen züchtet, bei denen das Genprodukt exprimiert wird; und das Genprodukt sammelt.

7. Verfahren nach Anspruch 6, wobei der Promotor in der Genexpressions-Einheit für das heterologe Genprodukt ein Drosophila-Promotor ist.

8. Verfahren nach Anspruch 7, wobei der Promotor der Metallothionein-Promotor, der Hsp-70-Promotor oder der 5'-LTR eines COPIA-Elements ist.

9. Verfahren nach einem der Ansprüche 6 bis 8, wobei die Genexpressions-Einheit für das heterologe Genprodukt und der Selektionsmarker in getrennten Vektoren liegen, mit denen die Drosophila-Zellen cotransfiziert werden.

10. Verfahren nach einem der Ansprüche 6 bis 9, wobei das heterologe Genprodukt ein Protein, das von einer für tPA codierenden DNA-Sequenz codiert wird, oder eine aktive Variante davon ist.

11. Verfahren zur Herstellung eines Proteins, das von einer für tPA codierenden DNA-Sequenz codiert wird, oder einer aktiven Variante davon in Drosophila-Zellen, bei dem man Zellen mit einem Vektor, enthaltend eine Genexpressions-Einheit für tPA und einem Vektor enthaltend einen selektierbaren Marker cotransfiziert, transfizierte Zellen unter Bedingungen züchtet, bei denen das tPA exprimiert und sezerniert wird, und das sezernierte tPA sammelt.

12. tPA-Genexpressions-Einheit, enthaltend eine für tPA oder eine aktive Variante davon codierende DNA-Sequenz, und ein Regulationselement, das in Drosophila-Zellen funktioniert und für die Transkription der tPA-codierenden Sequenz und die anschließende Translation in einer Drosophila-Zelle erforderlich ist.

13. Verfahren zur Expression eines heterologen Genprodukts in Drosophila-Zellen, bei dem man die Zellen mit einer Genexpressions-Einheit für das heterologe Genprodukt und einem DHFR-Selektionsmarker cotransfiziert; die transfizierten Zellen züchtet, wodurch das DHFR-Gen und die heterologe Genexpressions-Einheit ohne weitere Amplifikation exprimiert werden; und das heterologe Genprodukt sammelt.

14. Verfahren nach Anspruch 13, wobei der Promotor der Genexpressions-Einheit für das heterologe Genprodukt ein Drosophila-Promotor ist.

**15.** Verfahren nach Anspruch 14, wobei der Promotor der Methallothionein-Promotor, der Hsp-70-Promotor oder der 5'-LTR eines COPIA-Elements ist.

**16.** Protein, das von einer DNA-Sequenz codiert wird, die für tPA oder eine aktive Variante davon codiert, erhältlich durch das Verfahren nach einem der Ansprüche 1, 6, 11 und 13.

**17.** Arzneimittel zur Bewirkung der Thrombolyse in einem Menschen, das eine wirksame, nicht-toxische Menge des tPA von Anspruch 16 und einen pharmazeutischen Träger dafür enthält.

**Patentansprüche für folgenden Vertragsstaat : ES**

**1.** Verfahren zur Expression eines nicht-bakteriellen, heterologen Genprodukts in Drosophila-Zellen, bei dem man die Zellen mit einer Genexpressions-Einheit für das heterologe Genprodukt und einem Selektionsmarker transfiziert; die transfizierten Zellen unter Bedingungen züchtet, bei denen das Genprodukt exprimiert wird; und das Genprodukt sammelt.

**2.** Verfahren nach Anspruch 1, wobei der Promotor in der Genexpressions-Einheit ein Drosophila-Metallothionein-Promotor ist.

**3.** Verfahren nach einem der vorhergehenden Ansprüche, wobei der Selektionsmarker die Hygromycin B-Phosphotransferase oder die Dihydrofolatreduktase (DHFR) ist.

**4.** Verfahren nach einem der vorhergehenden Ansprüche, wobei die Genexpressions-Einheit für das heterologe Genprodukt und der Selektionsmarker in getrennten Vektoren liegen, mit denen die Drosophila-Zellen cotransfiziert werden.

**5.** Verfahren nach einem der vorhergehenden Ansprüche, wobei das heterologe Genprodukt ein Protein, das von einer für tPA codierenden DNA-Sequenz codiert wird, oder eine aktive Variante davon ist.

**6.** Verfahren zur Expression eines heterologen Genprodukts in Drosophila-Zellen, bei dem man die Zellen mit einer Genexpressions-Einheit für das heterologe Genprodukt und einem Selektionsmarker transfiziert, wobei der Selektionsmarker die Hygromycin B-Phosphotransferase ist; die transfizierten Zellen unter Bedingungen züchtet, bei denen das Genprodukt exprimiert wird; und das Genprodukt sammelt.

**7.** Verfahren nach Anspruch 6, wobei der Promotor in der Genexpressions-Einheit für das heterologe Genprodukt ein Drosophila-Promotor ist.

**8.** Verfahren nach Anspruch 7, wobei der Promotor der Metallothionein-Promotor, der Hsp-70-Promotor oder der 5'-LTR eines COPIA-Elements ist.

**9.** Verfahren nach einem der Ansprüche 6 bis 8, wobei die Genexpressions-Einheit für das heterologe Genprodukt und der Selektionsmarker in getrennten Vektoren liegen, mit denen die Drosophila-Zellen cotransfiziert werden.

**10.** Verfahren nach einem der Ansprüche 6 bis 9, wobei das heterologe Genprodukt ein Protein, das von einer für tPA codierenden DNA-Sequenz codiert wird, oder eine aktive Variante davon ist.

**11.** Verfahren zur Herstellung eines Proteins, das von einer tPA-codierenden DNA-Sequenz codiert wird oder einer aktiven Variante davon in Drosophila-Zellen, bei dem man Zellen mit einem Vektor, enthaltend eine Genexpressions-Einheit für tPA und einem Vektor enthaltend einen selektierbaren Marker cotransfiziert, transfizierte Zellen unter Bedingungen züchtet, bei denen das tPA exprimiert und sezerniert wird, und das sezernierte tPA sammelt.

**12.** Verfahren zur Expression eines heterologen Genprodukts in Drosophila-Zellen, bei dem man die Zellen mit einer Genexpressions-Einheit für das heterologe Genprodukt und einem DHFR-Selektionsmarker cotransfiziert; die transfizierten Zellen züchtet, wodurch das DHFR-Gen und die heterologe Genexpressions-Einheit ohne weitere Amplifikation exprimiert werden; und das heterologe Genprodukt sammelt.

**13.** Verfahren nach Anspruch 13, wobei der Promotor in der Genexpressionseinheit für das heterologe Genprodukt ein Drosophila-Promotor ist.

**14.** Verfahren nach Anspruch 13, wobei der Promotor der Methallothionein-Promotor, der Hsp-70-Promotor oder der 5'-LTR eines COPIA-Elements ist.

**Revendications**
**Revendications pour les Etats contractants suivants : AT, BE, CH, DE, FR, GB, IT, LI, LU, NL, SE, GR**

**1.** Méthode pour exprimer un produit génique hétérologue non-bactérien dans des cellules de Drosophila, qui comprend la transfection de ces cellules avec une unité d'expression génique pour le produit génique hétérologue et un marqueur de sélection ; la culture des cellules transfectées dans des conditions telles que le produit génique est exprimé ; et la collection du produit génique.

**2.** Méthode suivant la revendication 1, dans laquelle le promoteur dans l'unité d'expression génique est un promoteur métallothionéine de Drosophila.

**3.** Méthode suivant l'une quelconque des revendications précédentes, dans laquelle le marqueur de sélection est l'hygromycine B phosphotransférase ou la dihydrofolate réductase (DHFR).

**4.** Méthode suivant l'une quelconque des revendications précédentes, dans laquelle l'unité d'expression génique pour le produit génique hétérologue et le marqueur de sélection sont dans des vecteurs séparés avec lesquels les cellules de Drosophila sont co-transfectées.

**5.** Méthode suivant l'une quelconque des revendications précédentes, dans laquelle le produit génique hétérologue est une protéine codée par une séquence codante d'ADN pour le tPA ou un variant actif de celui-ci.

**6.** Méthode pour exprimer un produit génique hétérologue dans des cellules de Drosophila, qui comprend la transfection des cellules avec une unité d'expression génique pour le produit génique hétérologue et un marqueur de sélection, dans laquelle le marqueur de sélection est l'hygromycine B phosphotransférase ; la culture des cellules transfectées dans des conditions telles que le produit génique est exprimé ; et la collection du produit génique.

**7.** Méthode suivant la revendication 6, dans laquelle le promoteur dans l'unité d'expression génique pour le produit génique hétérologue est un promoteur de Drosophila.

**8.** Méthode suivant la revendication 7, dans laquelle le promoteur est le promoteur métallothionéine, le promoteur Hsp-70 ou le 5' LTR d'un élément COPIA.

**9.** Méthode suivant l'une quelconque des revendications 6 à 8, dans laquelle l'unité d'expression génique pour le produit génique hétérologue et le marqueur de sélection sont dans des vecteurs séparés avec lesquels les cellules de Drosophila sont co-transfectées.

**10.** Méthode suivant l'une quelconque des revendications 6 à 9, dans laquelle produit génique hétérologue est une protéine codée par une séquence codante d'ADN pour le tPA ou un variant actif de celui-ci.

**11.** Méthode pour la production d'une protéine codée par une séquence codante d'ADN pour le tPA ou un variant actif de celui-ci dans des cellules Drosophila, qui comprend la co-transfection des cellules avec un vecteur contenant une unité d'expression génique pour le tPA et un vecteur contenant un marqueur pouvant être sélectionné, la culture des cellules transfectées dans des conditions telles que le tPA est exprimé et sécrété et la collection du tPA sécrété.

**12.** Unité d'expression génique de tPA comprenant une séquence codante d'ADN pour le tPA, ou un variant actif de celui-ci, et un élément régulateur qui fonctionne dans des cellules de Drosophila nécessaires pour la transcription de la séquence codante pour le tPA et la traduction ultérieure à l'intérieur d'une cellule de Drosophila.

**13.** Méthode pour exprimer un produit génique hétérologue dans des cellules de Drosophila qui comprend la co-transfection de ces cellules avec une unité d'expression-génique pour le produit génique hétérologue et un marqueur de sélection DHFR ; la culture des cellules transfectées de façon à exprimer le gène DHFR et l'unité d'expression hétérologue sans amplification supplémentaire ; et la collection du produit génique hétérologue.

**14.** Méthode suivant la revendication 13, dans laquelle le promoteur dans l'unité d'expression génique pour le produit génique hétérologue est un promoteur de Drosophila.

**15.** Méthode suivant la revendication 14, dans laquelle le promoteur est le promoteur métallothionéine, le promoteur Hsp-70 ou le 5' LTR d'un élément COPIA.

**16.** Protéine codée par une séquence codante d'ADN pour le tPA ou un variant actif de celui-ci pouvant être obtenue par la méthode suivant l'une quelconque des revendications 1, 6, 11 et 13.

**17.** Composition pharmaceutique pour effectuer la thrombolyse chez un sujet humain, qui comprend une quantité efficace, non-toxique, du tPA suivant la revendication 16 et un support pharmaceutique pour celui-ci.

**Revendications pour l'Etat contractant suivant : ES**

**1.** Méthode pour exprimer un produit génique hétérologue non-bactérien dans des cellules de Drosophila, qui comprend la transfection de ces cellules avec une unité d'expression génique pour le produit génique hétérologue et un marqueur de sélection ; la culture des cellules transfectées dans des conditions telles que le produit génique est exprimé ; et la collection du produit génique.

**2.** Méthode suivant la revendication 1, dans laquelle le promoteur dans l'unité d'expression génique est un promoteur métallothionéine de Drosophila.

**3.** Méthode suivant l'une quelconque des revendications précédentes, dans laquelle le marqueur de sélection est l'hygromycine B phosphotransférase ou la dihydrofolate réductase (DHFR).

**4.** Méthode suivant l'une quelconque des revendications précédentes, dans laquelle l'unité d'expression génique pour le produit génique hétérologue et le marqueur de sélection sont dans des vecteurs séparés avec lesquels les cellules de Drosophila sont co-transfectées.

**5.** Méthode suivant l'une quelconque des revendications précédentes, dans laquelle le produit génique hétérologue est une protéine codée par une séquence codante d'ADN pour le tPA ou un variant actif de celui-ci.

**6.** Méthode pour exprimer un produit génique hétérologue dans des cellules de Drosophila, qui comprend la transfection des cellules avec une unité d'expression génique pour le produit génique hétérologue et un marqueur de sélection, dans laquelle le marqueur de sélection est l'hygromycine B phosphotransférase ; la culture des cellules transfectées dans des conditions telles que le produit génique est exprimé ; et la collection du produit génique.

**7.** Méthode suivant la revendication 6, dans laquelle le promoteur dans l'unité d'expression génique pour le produit génique hétérologue est un promoteur de Drosophila.

**8.** Méthode suivant la revendication 7, dans laquelle le promoteur est le promoteur métallothionéine, le promoteur Hsp-70 ou le 5' LTR d'un élément COPIA.

**9.** Méthode suivant l'une quelconque des revendications 6 à 8, dans laquelle l'unité d'expression génique pour le produit génique hétérologue et le marqueur de sélection sont dans des vecteurs séparés avec lesquels les cellules de Drosophila sont co-transfectées.

**10.** Méthode suivant l'une quelconque des revendications 6 à 9, dans laquelle produit génique hétérologue est une protéine codée par une séquence codante d'ADN pour le tPA ou un variant actif de celui-ci.

**11.** Méthode pour la production d'une protéine codée par une séquence codante d'ADN pour le tPA ou un variant actif de celui-ci dans des cellules Drosophila, qui comprend la co-transfection des cellules avec un vecteur contenant une unité d'expression génique pour le tPA et un vecteur contenant un marqueur pouvant être sélectionné, la culture des cellules transfectées dans des conditions telles que le tPA est exprimé et sécrété et la collection du tPA sécrété.

**12.** Méthode pour exprimer un produit génique hétérologue dans des cellules de Drosophila, qui comprend la co-transfection de ces cellules avec une unité d'expression génique pour le produit génique hétérologue et un marqueur de sélection DHFR ; la culture des cellules transfectées, de façon à exprimer le gène DHFR et l'unité d'expression génique hétérologue sans amplification supplémentaire ; et la collection du produit génique hétérologue.

**13.** Méthode suivant la revendication 12, dans laquelle le promoteur dans l'unité d'expression génique pour le produit génique hétérologue est un promoteur de Drosophila.

**14.** Méthode suivant la revendication 13, dans laquelle le promoteur est le promoteur métallothionéine, le promoteur Hsp-70 ou le 5' LTR d'un élément COPIA.